# EUROPÄISCHE PATENTSCHRIFT

(11) **EP 0 586 975 B1**
(45) Veröffentlichungstag und Bekanntmachung des Hinweises auf die Patenterteilung: **10.04.1996**
(21) Anmeldenummer: 93113653.5
(22) Anmeldetag: 26.08.1993
(51) Int. Cl.: C07C 303/44, C07C 303/06, C07C 309/47

(54) **Verfahren zur Isolierung von 2-Naphthylamin-1,5,7,-trisulfonsäure**
Method for the isolation of 2-naphthylamine-1,5,7,-trisulphonic acid
Procédé d'isolement de l'acide 2-naphtylamine-1,5,7,-trisulfonique

(30) Priorität: 08.09.1992 DE 4229997
(43) Veröffentlichungstag der Anmeldung: 16.03.1994
(73) Patentinhaber: BAYER AG, D-51368 Leverkusen (DE)
(72) Erfinder: Holger, Heidenreich Dr., D-25712 Kuden (DE); Horst, Behre Dr., D-51519 Odenthal (DE)

(56) Entgegenhaltungen:
- EP-A- 0 074 024

## Beschreibung

Die Erfindung betrifft die Isolierung von 2-Napthylamin-1.5.7-trisulfonsäure ("Sulfo-Amino-I-Säure") aus diese Säure enthaltenden Sulfiergemischen in verbesserter Qualität.

2-Naphthylamin-1.5.7-trisulfonsäure ist ein wichtiges Produkt für die Herstellung von Farbstoffen, insbesondere von Reaktivfarbstoffen. Sie kann aus 2-Naphthylamin-1-sulfonsäure ("Tobiassäure") durch Sulfierung in rauchender Schwefelsäure ("Oleum") erhalten werden (siehe DE-OS 28 42 948).

Aus den entstehenden Reaktionsgemischen kann man die 2-Naphthylamin-1.5.7-trisulfonsäure durch Verdünnen mit Eiswasser bzw. Eiswasser in Verbindung mit Alkalimetallsalzen als freie Säure bzw. deren Salz bei Temperaturen unterhalb von 40°C fällen (siehe CS-PS 162 194). Allerdings ist die Isolierausbeute an 2-Naphthylamin-1.5.7-trisulfonsäure nur gering (<30 % s. A. Kraslawski et al., Zesz.Nauk.-Politech.Lodz., Inz. Chem. 1983, 407 (11), 87-95).

Meistens wird die 2-Naphthylamin-1.5.7-trisulfonsäure wegen der leichten Hydrolysierbarkeit der Sulfonsäuregruppe in 1-Stellung als reaktive Zwischenstufe zur Herstellung der 2-Naphthylamin-5.7-disulfonsäure lediglich durchlaufen (s. US-PS 4 198 529; CS-PS 170 749).

Die Abscheidung der 2-Naphthylamin-1.5.7-trisulfonsäure als freie Säure oder als Alkalisalz ist mit dem Nachteil verbunden, daß die Sulfo-Amino-I-Säure durch Zersetzungsprodukte wie 2-Naphthylamin-5.7-disulfonsäure und 2-Naphthylamin-5-sulfonsäure verunreinigt ist (siehe CS-PS 170.749) und in einer Kristallform ausfällt, in der sie erhebliche Mengen (20 bis 30 Gew.-%) Schwefelsäure enthalt. Dieser Schwefelsäuregehalt der abgetrennten 2-Naphthylamin-1.5.7-trisulfonsäure läßt sich durch Waschen mit Wasser oder stark verdünnter Schwefelsäure infolge der hohen Löslichkeit der 2-Naphthylamin-1.5.7-trisulfonsäure in Wasser und verdünnter Schwefelsäure nur unter hohen Ausbeuteverlusten vermindern. Die Ausfällung der 2-Naphthylamin-1.5.7-trisulfonsäure mit Hilfe von Alkalisalzen ist mit dem Nachteil verbunden, daß dabei eine stark salzhaltige Dünnsäure anfällt, deren Aufarbeitung große technische Schwierigkeiten bereitet. Außerdem enthält die so gewonnene 2-Naphthylamin-1.5.7-trisulfonsäure meist größere Mengen (bis zu 15 Gew.-%) Natriumsulfat bzw. Natriumchlorid und anhaftende Restsäure (bis zu 30 Gew.-%), die, da diese Verunreinigungen bei der Weiterverarbeitung stören, durch Waschen mit verdünnter Schwefelsäure und Wasser entfernt werden müssen. Dieses Waschen ist aber mit erheblichen Ausbeuteverlusten verbunden.

Es wurde nun gefunden, daß man die bei der Ausfällung der 2-Naphthylamin-1.5.7-trisulfonsäure auftretenden Schwierigkeiten dadurch vermeiden kann, daß man die Ausfällung durch Verdünnen der Reaktionsgemische unter exakt definierten Bedingungen vornimmt.

Gegenstand der Erfindung ist ein Verfahren zur Isolierung von 2-Naphthylamin-1.5.7-trisulfonsäure aus einem Reaktionsgemisch, das, bezogen auf das gesamte Reaktionsgemisch, 15 bis 40, vorzugsweise 25 bis 35 Gew.-% 2-Naphthylamin-1.5.7-trisulfonsäure und 60 bis 85, vorzugsweise 65 bis 75 Gew.-% Schwefelsäure oder Oleum mit einem SO₃-Gehalt, bezogen auf Schwefelsäure + SO₃, von 1 bis 25, vorzugsweise 15 bis 20 Gew.-% enthält, durch (a) Verdünnen mit verdünnter Schwefelsäure und Wasser auf einen Schwefelsäuregehalt, bezogen auf Schwefelsäure und Wasser, von 20 bis 80, vorzugsweise 35 bis 60, besonders bevorzugt 40 bis 55 und insbesondere 45 bis 51 Gew.-% unter (b) Aufrechterhaltung einer Temperatur von 30 bis 80, vorzugsweise 35 bis 70, besonders bevorzugt von 45 bis 65 und insbesondere von 54 bis 59°C in einer Weise, daß (i) das Verhältnis von zugesetzter zu ursprünglich vorhandener Schwefelsäure - berechnet als 100 %ige Schwefelsäure - mindestens 0,1, vorzugsweise mindestens 0,2, insbesondere mindestens 0,4 beträgt und (ii) der Schwefelsäuregehalt während des gesamten Verdünnungsvorgangs - ideale Vermischung vorausgesetzt - die Untergrenze des Bereichs (a) um nicht mehr als 10 % unterschreitet.

Wesentlich für das Erzielen des erfindungsgemäßen Effekts ist also - neben der Temperatur - nicht nur der Schwefelsäuregehalt am Ende des Verfahrens, sondern offenbar auch das Vermeiden von temporär zu niedrigen Schwefelsäurekonzentrationen, wie sie z.B. beim Einlaufenlassen des Reaktionsgemisches in Wasser durchlaufen werden. Erfindungsgemäß arbeitet man also vorzugsweise so, daß vom Schwefelsäuregehalt des Reaktionsgemisches aus betrachtet beim Verdünnen möglichst kein Minimum oder nur ein solches Minimum durchschritten wird, das dem Schwefelsäuregehalt-Endwert eng benachbart ist.

Grundsätzlich kann das erfindungsgemäße Verfahren so ausgeführt werden, daß man das zu verdünnende Reaktionsgemisch und Wasser in verdünnte Schwefelsäure einer Konzentration gibt, die wenigstens den anspruchsgemäß geforderten Schwefelsäuremindestgehalt besitzt.

Nach einer bevorzugten Ausführungsform werden Reaktionsgemisch, Wasser und verdünnte Schwefelsäure gleichzeitig so dosiert, daß sich die gewünschte Konzentration einstellt und während des gesamten Mischungsvorgangs praktisch konstant gehalten wird.

Anstelle frischer verdünnter Schwefelsäure kann zum Verdünnen auch die nach dem Ausfällen der 2-Naphthylamin-1.5.7-trisulfonsäure verbleibende Schwefelsäure ("Muttersäure") eines vorhergehenden Ansatzes verwendet werden. Solche "Muttersäure" enthält, bezogen auf die Summe von Wasser und Schwefelsäure, im allgemeinen 20 bis 80, vorzugsweise 35 bis 60, besonders bevorzugt 40 bis 55 und insbesondere 45 bis 51 Gew.-% Schwefelsäure und, bezogen auf Schwefelsäure 0,1 bis 3,0, vorzugsweise 0,5 bis 1,5 Gew.-% 2-Naphthylamin-1.5.7-trisulfonsäure, 0,1 bis 2,5, vorzugsweise 0,5 bis 1,5 Gew.-% 2-Naphthylamin-5.7-disulfonsäure, 0,1 bis 1,5, vorzugsweise 0,3 bis 1,0 Gew.-% 2-Naphthylamin-6.8-disulfonsäure, 0,1 bis 1,0, vorzugsweise 0,2 bis 0,5 Gew.-% 2-Naphthylamin-3.5-disulfonsäure, 0,1 bis 1,0, vorzugsweise 0,2 bis 0,4 Gew.-% 2-Naphthylamin-3.5.7-trisulfonsäure und 0,1 bis 0,5, vorzugsweise 0,2 bis 0,4 Gew.-% 2-Naphthylamin-3.6.8-trisulfonsäure. Es zeigt sich, daß die Verwendung von Muttersäure anstelle reiner verdünnter Schwefelsäure zu einer saubereren Ware, zu besserer Filtrierbarkeit und zu einem geringeren Schwefelsäuregehalt des isolierten Produkts führt.

Die beim Verdünnungsvorgang anfallende Wärme wird durch entsprechende Kühlung abgeführt.

Bei dem erfindungsgemäßen Verfahren fällt die 2-Naphthylamin-1.5.7-trisulfonsäure in einer gut filtrierbaren Form mit hoher Isomerenreinheit an; der Gehalt an 2-Naphthylamin-1.5.7-trisulfonsäure liegt im Bereich oberhalb von 55 Gew.-%, bezogen auf feuchtes Produkt (oberhalb von 70 Gew.-%, umgerechnet auf trockenes Produkt), und der Gehalt an Schwefelsäure, bezogen auf feuchtes Produkt, liegt zwischen 12 und 20 Gew.-% (berechnet als 100 %ige H₂SO₄) (zwischen 16 und 27 Gew.-%, umgerechnet auf trockenes Produkt).

Es hat sich als vorteilhaft erwiesen, die ausgefällte 2-Naphthylamin-1.5.7-trisulfonsäure noch 0,1 bis 10, vorzugsweise 0,2 bis 5, insbesondere 0,5 bis 2 Stunden bei Temperaturen von 20 bis 55°C, vorzugsweise bei 30 bis 45°C in der verdünnten Lösung zu belassen, bevor man das Produkt abtrennt.

Das erfindungsgemäße Verfahren kann diskontinuierlich und kontinuierlich ausgeführt werden.

Die Prozentangaben in den nachfolgenden Beispielen beziehen sich auf das Gewicht.

### Beispiele

### Beispiel 1

300 g Tobiassäure werden mit 1240 g Oleum (25 % SO₃-Gehalt), unter sofortiger Abführung der Reaktionswärme bei einer Temperatur von 18 bis 20°C sulfiert. Nach einer Verweilzeit von 1 Stunde bei 20°C dosiert man anschließend 188 g Oleum (65 % SO₃-Gehalt) hinzu und erwärmt auf 110°C. Nach 4 Stunden wird das Reaktionsgemisch simultan und kontinuierlich mit 1634 g Wasser und mit 634 g rückgeführter Muttersäure aus einem vorigen Ansatz bei einer Temperatur von 58°C verdünnt. Nach einer Verweilzeit von 1 Stunde bei 58°C wird die partiell ausgefällte Suspension in einen weiteren Rührbehälter abgelassen und innerhalb einer Stunde auf ca. 40°C abgekühlt. Nach einer Rührzeit von weiteren 23 Stunden wird die Suspension filtriert. Man erhält 669 g eines gelbgrauen Filterkuchens der folgenden Zusammensetzung:

| | |
|---|---|
| 2-Naphthylamin-1.5.7-trisulfonsäure | 57,56 % |
| 2-Naphthylamin-1.3.5-trisulfonsäure | 0,24 % |
| 2-Naphthylamin-3.5.7-trisulfonsäure | 0,22 % |
| 2-Naphthylamin-3.6.8-trisulfonsäure | 0,22 % |
| 2-Naphthylamin-5.7-disulfonsäure | 0,96 % |
| 2-Naphthylamin-6.8-disulfonsäure | 1,06 % |
| 2-Naphthylamin-1.5-disulfonsäure | 0,09 % |
| 2-Naphthylamin-3.5-disulfonsäure | 0,65 % |
| Schwefelsäure | 15,9 % |
| Wasser | 23,1 % |

### Beispiel 2

300 g Tobiassäure werden mit 1240 g Oleum (25 % SO₃-Gehalt) unter sofortiger Abführung der Reaktionswärme bei einer Temperatur von 18 bis 20°C sulfiert. Nach einer Verweilzeit von 1 Stunde bei 20°C dosiert man anschließend 188 g Oleum (65 % SO₃-Gehalt) hinzu und erwärmt auf 110°C. Nach 4 Stunden wird das Reaktionsgemisch simultan und kontinuierlich mit 1634 g Wasser und mit 634 g 50 %iger Schwefelsäure bei einer Temperatur von 58°C verdünnt. Nach einer Verweilzeit von 1 Stunde bei 58°C wird die partiell ausgefällte Suspension in einen weiteren Rührbehälter abgelassen und innerhalb einer Stunde auf ca. 40°C abgekühlt. Nach einer Rührzeit von weiteren 23 Stunden wird die Suspension filtriert. Man erhält 669 g eines gelbbraunen Filterkuchens der folgenden Zusammensetzung:

| | |
|---|---|
| 2-Naphthylamin-1.5.7-trisulfonsäure | 50,94 % |
| 2-Naphthylamin-1.3.5-trisulfonsäure | 0,25 % |
| 2-Naphthylamin-3.5.7-trisulfonsäure | 0,24 % |
| 2-Naphthylamin-3.6.8-trisulfonsäure | 0,22 % |
| 2-Naphthylamin-5.7-disulfonsäure | 0,98 % |
| 2-Naphthylamin-6.8-disulfonsäure | 0,34 % |
| 2-Naphthylamin-1.5-disulfonsäure | 0,19 % |
| 2-Naphthylamin-3.5-disulfonsäure | 0,44 % |
| Schwefelsäure | 18,9 % |
| Wasser | 27,5 % |

### Beispiel 3 (Vergleich)

300 g Tobiassäure werden mit 1240 g Oleum (25 % SO₃-Gehalt) unter sofortiger Abführung der Reaktionswärme bei einer Temperatur von 18 bis 20°C sulfiert. Nach einer Verweilzeit von 1 Stunde bei 20°C dosiert man anschließend 188 g Oleum (65 % SO₃-Gehalt) hinzu und erwärmt auf 110°C. Nach 4 Stunden wird das Reaktionsgemisch simultan und kontinuierlich mit 1634 g Wasser bei einer Temperatur von 25°C verdünnt. Nach einer Verweilzeit von 1 Stunde bei 25°C wird die partiell ausgefällte Suspension in einen weiteren Rührbehälter abgelassen und innerhalb einer Stunde auf ca. 20°C abgekühlt. Nach einer Rührzeit von weiteren 23 Stunden wird die Suspension filtriert. Man erhält 855 g eines gelblichfeuchten Filterkuchens der folgenden Zusammensetzung:

| | |
|---|---|
| 2-Naphthylamin-1.5.7-trisulfonsäure | 44,64 % |
| 2-Naphthylamin-1.3.5-trisulfonsäure | 0,14 % |
| 2-Naphthylamin-3.5.7-trisulfonsäure | 0,18 % |
| 2-Naphthylamin-3.6.8-trisulfonsäure | 0,12 % |
| 2-Naphthylamin-5.7-disulfonsäure | 1,09 % |
| 2-Naphthylamin-6.8-disulfonsäure | 0,27 % |
| 2-Naphthylamin-1.5-disulfonsäure | 0,01 % |
| 2-Naphthylamin-3.5-disulfonsäure | 0,35 % |
| Schwefelsäure | 21,9 % |
| Wasser | 31,3 % |

## Patentansprüche

1. Verfahren zur Isolierung von 2-Naphthylamin-1.5.7-trisulfonsäure aus einem Reaktionsgemisch, das, bezogen auf das gesamte Reaktionsgemisch, 15 bis 40 Gew.-% 2-Naphthylamin-1.5.7-trisulfonsäure und 60 bis 85 Gew.-% Schwefelsäure oder Oleum mit einem SO₃-Gehalt, bezogen auf Schwefelsäure + SO₃, von 1 bis 25 Gew.-% enthält, durch (a) Verdünnen mit verdünnter Schwefelsäure und Wasser auf einen Schwefelsäuregehalt, bezogen auf Schwefelsäure und Wasser, von 20 bis 80 Gew.-% unter (b) Aufrechterhaltung einer Temperatur von 30 bis 80°C in einer Weise, daß (i) das Verhältnis von zugesetzter zu ursprünglich vorhandener Schwefelsäure - berechnet als 100 %ige Schwefelsäure - mindestens 0,1 beträgt und (ii) der Schwefelsäuregehalt während des gesamten Verdünnungsvorgangs - ideale Vermischung vorausgesetzt - die Untergrenze des Bereichs (a) um nicht mehr als 10 % unterschreitet.

2. Verfahren nach Anspruch 1, wonach ein Reaktionsgemisch mit einem Gehalt von 25 bis 35 Gew.-% 2-Naphthylamin-1.5.7-trisulfonsäure und 65 bis 75 Gew.-% Schwefelsäure oder Oleum eingesetzt wird.

3. Verfahren nach Anspruch 1, wonach das Oleum einen SO₃-Gehalt von 15 bis 20 Gew.-% besitzt.

4. Verfahren nach Anspruch 1, wonach man auf einen Schwefelsäuregehalt von 35 bis 60 Gew.-% verdünnt.

5. Verfahren nach Anspruch 1, wonach man auf einen Schwefelsäuregehalt von 40 bis 55 Gew.-% verdünnt.

6. Verfahren nach Anspruch 1, wonach man auf einen Schwefelsäuregehalt von 45 bis 51 Gew.-% verdünnt.

7. Verfahren nach Anspruch 1, wonach man in einem Temperaturbereich von 35 bis 70°C arbeitet.

8. Verfahren nach Anspruch 1, wonach das Verhältnis (i) mindestens 0,2 beträgt.

9. Verfahren nach Anspruch 1, wonach man gleichzeitig mit Wasser und verdünnter Schwefelsäure verdünnt.

10. Verfahren nach Anspruch 1, wonach man anstelle von verdünnter Schwefelsäure Muttersäure eines vorangegangenen Ansatzes verwendet.

## Claims

1. Process for the isolation of 2-naphthylamine-1,5,7-trisulphonic acid from a reaction mixture which contains, based on the entire reaction mixture, 15 to 40% by weight of 2-naphthylamine-1,5,7-trisulphonic acid and 60 to 85% by weight of sulphuric acid or oleum with an SO₃ content, based on sulphuric acid + SO₃, of 1 to 25% by weight, by (a) dilution with dilute sulphuric acid and water to a sulphuric acid content, based on sulphuric acid and water, of 20 to 80% by weight, while (b) maintaining a temperature of 30 to 80°C, in such a way that (i) the ratio of sulphuric acid added to sulphuric acid originally present - calculated as 100% strength sulphuric acid - is at least 0.1, and (ii) the sulphuric acid content throughout the dilution process - assuming ideal mixing - falls short of the lower limit of the range (a) by not more than 10%.

2. Process according to Claim 1, wherein a reaction mixture containing 25 to 35% by weight of 2-naphthylamine-1,5,7-trisulphonic acid and 65 to 75% by weight of sulphuric acid or oleum is used.

3. Process according to Claim 1, wherein the oleum has an SO₃ content of 15 to 20% by weight.

4. Process according to Claim 1, wherein dilution is carried out to a sulphuric acid content of 35 to 60% by weight.

5. Process according to Claim 1, wherein dilution is carried out to a sulphuric acid content of 40 to 55% by weight.

6. Process according to Claim 1, wherein dilution is carried out to a sulphuric acid content of 45 to 51% by weight.

7. Process according to Claim 1, wherein the operating temperature is in the range from 35 to 70°C.

8. Process according to Claim 1, wherein the ratio (i) is at least 0.2.

9. Process according to Claim 1, wherein dilution is carried out simultaneously with water and dilute sulphuric acid.

10. Process according to Claim 1, wherein mother acid from a previous batch is used instead of dilute sulphuric acid.

## Revendications

1. Procédé d'isolement d'acide 2-naphtylamine-1,5,7-trisulfonique à partir d'un mélange réactionnel qui contient, par rapport au mélange réactionnel global, 15 à 40 % en poids d'acide 2-naphtylamine-1,5,7-trisulfonique et 60 à 85 % en poids d'acide sulfurique ou d'oléum ayant une teneur en SO₃, par rapport à la somme acide sulfurique + SO₃, de 1 à 25 % en poids, par (a) dilution avec de l'acide sulfurique dilué et de l'eau à une teneur en acide sulfurique, rapportée à la somme de l'acide sulfurique et de l'eau, de 20 à 80 % en poids en (b) maintenant une température de 30 à 80°C de telle sorte que (i) le rapport de l'acide sulfurique ajouté à l'acide sulfurique présent initialement - calculé sous forme d'acide sulfurique à 100 % - est d'au moins 0,1 et (ii) la teneur en acide sulfurique pendant tout le processus de dilution - en supposant qu'il y ait un mélange idéal - ne dépasse pas la limite inférieure du domaine (a) de plus de 10 % vers les concentrations inférieures.

2. Procédé selon la revendication 1, selon lequel on utilise un mélange réactionnel ayant une teneur de 25 à 35 % en poids d'acide 2-naphtylamine-1.5.7-trisulfonique et 65 à 75 % en poids d'acide sulfurique ou d'oléum.

3. Procédé selon la revendication 1, selon lequel l'oléum possède une teneur en SO₃ de 15 à 20 % en poids.

4. Procédé selon la revendication 1, selon lequel on dilue à une teneur en acide sulfurique de 35 à 60 % en poids.

5. Procédé selon la revendication 1, selon lequel on dilue à une teneur en acide sulfurique de 40 à 55 % en poids.

6. Procédé selon la revendication 1, selon lequel on dilue à une teneur en acide sulfurique de 45 à 51 % en poids.

7. Procédé selon la revendication 1, selon lequel on travaille dans un intervalle de température de 35 à 70°C.

8. Procédé selon la revendication 1, selon lequel le rapport (i) est d'au moins 0,2.

9. Procédé selon la revendication 1, selon lequel on dilue simultanément avec de l'eau et de l'acide sulfurique dilué.

10. Procédé selon la revendication 1, selon lequel au lieu d'acide sulfurique dilué on utilise la liqueur mère acide d'un lot de fabrication antérieur.
